(19) Europäisches Patentamt
European Patent Office
Office européen des brevets

(11) **EP 3 578 102 B1**

(12) **EUROPÄISCHE PATENTSCHRIFT**

(45) Veröffentlichungstag und Bekanntmachung des Hinweises auf die Patenterteilung:
**19.05.2021 Patentblatt 2021/20**

(51) Int Cl.:
*A61B 6/00* *(2006.01)* *G06T 7/00* *(2017.01)*
*G06T 7/254* *(2017.01)* *G06T 5/50* *(2006.01)*
*A61B 6/12* *(2006.01)*

(21) Anmeldenummer: **18176498.6**

(22) Anmeldetag: **07.06.2018**

(54) **VERFAHREN ZUM BETREIBEN EINES MEDIZINISCHEN RÖNTGENGERÄTES; SOWIE RÖNTGENGERÄT**

METHOD FOR OPERATING A MEDICAL X-RAY DEVICE AND X-RAY DEVICE

PROCÉDÉ DE FONCTIONNEMENT D'UN APPAREIL À RAYONS X MÉDICAL ET APPAREIL À RAYONS X

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**11.12.2019 Patentblatt 2019/50**

(73) Patentinhaber: **Siemens Healthcare GmbH**
**91052 Erlangen (DE)**

(72) Erfinder: **Stowasser, Boris**
**91056 Erlangen (DE)**

(56) Entgegenhaltungen:
JP-A- 2010 279 594    US-A1- 2008 137 935
US-A1- 2009 076 369    US-A1- 2009 202 129
US-A1- 2010 142 792    US-B1- 6 314 160

**Beschreibung**

Die Erfindung betrifft ein Verfahren zum Betreiben eines medizinischen Röntgengerätes. Ein zweiter Aspekt der Erfindung betrifft ein medizinisches Röntgengerät.

Aus dem Stand der Technik bekannt sind Verfahren zum Durchführen einer Röntgenuntersuchung, insbesondere einer Fluoroskopie, bei welchen nacheinander mehrere Röntgenbilder aufgenommen werden, um eine zeitliche Veränderung an einer zu untersuchenden Körperregion zu erfassen. Eine solche zeitliche Veränderung betrifft insbesondere eine Bewegung an der Körperregion. Beispielsweise kann eine solche Bewegung durch die Zugabe eines Kontrastmittels in eine Körperflüssigkeit, welche die Körperregion durchströmt, gegeben sein. Mit anderen Worten kann das Kontrastmittel in der zu untersuchenden Körperregion injiziert werden und anhand der mehreren Röntgenbilder eine Ausbreitung des Kontrastmittels untersucht werden. Das Kontrastmittel muss hierzu derart gewählt sein, dass es in den Röntgenbildern sichtbar ist. Beispielsweise wird das Kontrastmittel in ein Gefäß, insbesondere ein Blutgefäß, der Körperregion injiziert. Mit anderen Worten kann hierbei ein Gefäßbaum der Körperregion sichtbar gemacht werden. Derartige Verfahren können unter dem Fachbegriff (digitale) Subtraktionsangiographie (DSA) zusammengefasst werden.

Alternativ kann die Bewegung eines medizinischen Objekts, beispielsweise eines Drahts (so genannte Guidewire) oder eines Katheters, durch die mehreren Röntgenbilder erfasst werden. Beispielsweise wird durch einen Anwender des medizinischen Röntgengerätes das medizinische Objekt an beziehungsweise in der Körperregion bewegt beziehungsweise platziert. Dieser Vorgang kann mittels der Röntgenuntersuchung überwacht werden. Derartige Verfahren werden auch mit dem Fachbegriff "Roadmap" bezeichnet.

Um eine bessere Sichtbarkeit des medizinischen Objekts beziehungsweise des Kontrastmittels zu erreichen, kann zunächst in einer ersten Phase der Röntgenuntersuchung ein so genanntes Maskenbild erstellt werden, in welcher die Körperregion statisch erfasst wird. Vorteilhafter Weise wird hierbei ein statisches Maskenbild der Körperregion erstellt, bevor die oben genannte Bewegung auftritt. Konkret kann diese erste Phase beziehungsweise das Aufnehmen des Maskenbildes stattfinden, bevor das Kontrastmittel injiziert wird oder das medizinische Objekt an die Körperregion bewegt wird.

In diesem Fall kann die Bewegung erst zu Beginn oder nach Beginn einer zweiten Phase der Röntgenuntersuchung erfolgen. In dieser zweiten Phase wird die Bewegung wie oben beschrieben durch die mehreren aufeinanderfolgenden Röntgenbildern visualisiert. Um eine verbesserte Übersicht über die Körperregion beziehungsweise die Bewegung zu ermöglichen, können jeweilige Gesamtbilder der zweiten Phase jeweils aus einem der mehreren Röntgenbilder und dem Maskenbild gebildet werden. Beispielsweise wird jedes der mehreren Röntgenbildern, welche im Rahmen der zweiten Phase aufgenommen werden, zusammen mit dem Maskenbild zu einem Gesamtbild weitergebildet. Da die Röntgenbilder der zweiten Phase nach den ersten Röntgenbildern beziehungsweise dem Maskenbild aufgenommen werden, werden diese vorliegend als zweite Röntgenbilder bezeichnet.

Beispielsweise kann ein jeweiliges Gesamtbild durch Subtraktion des Maskenbildes von einem jeweiligen der zweiten Röntgenbilder gebildet werden. Mit anderen Worten kann im Rahmen der Röntgenuntersuchung das Maskenbild von jedem der zweiten Röntgenbilder subtrahiert werden. Durch Subtraktion des Maskenbildes kann ein statischer Anteil der Bildinformation aus dem jeweiligen zweiten Röntgenbilder entfernt werden, was die Bewegung noch besser sichtbar macht.

Bei dem Aufnehmen von Röntgenbildern kann stets ein statistisches Bildrauschen auftreten. Dieses Bildrauschen kann mathematisch durch eine Varianz beschrieben werden. Das Bildrauschen beziehungsweise die Varianz fällt umso mehr ins Gewicht, je geringer eine Röntgendosis beim Aufnehmen des jeweiligen Röntgenbildes ist. Insbesondere wird ein Signal-zu-Rauschverhältnis mit sinkender Dosis immer schlechter. Es ergibt sich somit ein Zielkonflikt zwischen möglichst hoher Bildqualität und möglichst geringer Dosis. Als vorteilhaft hat sich hierbei erwiesen, mehrere Röntgenbilder in der ersten Phase aufzunehmen und diese arithmetisch zu dem Maskenbild zu mitteln. Mathematisch betrachtet ist hierbei die Varianz des gemittelten Maskenbildes indirekt proportional zur Anzahl an Röntgenbildern, über die gemittelt wird.

In der zweiten Phase der Röntgenuntersuchung ist in Mitteln über mehrere Röntgenbilder jedoch nicht ohne weiteres sinnvoll, da durch das Mitteln im Falle einer Bewegung der Kontrast reduziert wird. Beispielsweise wird die Bewegung aus den zweiten Röntgenbildern herausgemittelt. Wird nun ein jeweiliges Gesamtbild aus einem jeweiligen zweiten Röntgenbild und dem Maskenbild gebildet, so ist die erreichbare Bildqualität durch das Rauschen des jeweiligen zweiten Röntgenbildes beschränkt. In dem genannten Beispiel der Subtraktionsbildgebung, bei welcher das Maskenbild von dem jeweiligen zweiten Röntgenbild subtrahiert wird, kann dies mathematisch beispielhaft erklärt werden. Im Falle der Subtraktion des Maskenbildes von dem jeweiligen zweiten Röntgenbild addieren sich die jeweiligen Varianzen des Maskenbildes und des jeweiligen zweiten Röntgenbildes. Praktisch bedeutet dies, durch das Bildrauschen des zweiten Röntgenbildes ist die maximal erreichbare Bildqualität beschränkt, egal wie gut das Rauschen aus dem Maskenbild heraus gemittelt wird.

In der Schrift US 2008/0137935 A1 ist eine Methode zur Subtraktionsbildgebung offenbart, wobei eine Frequenzfiltermethode gemäß der in der Schrift US 5091925 A offenbarten Methode vorgeschlagen wird. In der Schrift US 2009/0076369 A1 wird eine Methode zur Erstellung eines zusammengesetzten Bildes mittels räumlicher Registrierung offenbart. Die Schrift US 6314160 B1 offenbart einen Algorithmus zur adaptiven fluoroskopischen Rauschreduzierung.

Es ist Aufgabe der vorliegenden Erfindung, die Bildqualität bei einer solchen Röntgenuntersuchung gegenüber dem Stand der Technik zu verbessern.

Diese Aufgabe wird erfindungsgemäß gelöst durch die Gegenstände der unabhängigen Patentansprüche. Vorteilhafte Ausführungsformen mit zweckmäßigen Weiterbildungen sind Gegenstand der Unteransprüche.

Ein erster Aspekt der Erfindung geht aus von einem Verfahren zum Betreiben eines medizinischen Röntgengerätes bei einer Röntgenuntersuchung, mit den Verfahrensschritten wie in Anspruch 1 definiert.

In dem Schritt a) werden mehrere erste Röntgenbilder für das Maskenbild aufgenommen. Diese werden zum Bilden des Maskenbildes gemäß einer Rekursionsformel gemittelt.

Das Aufnehmen der ersten Röntgenbilder kann als erste Phase der Röntgenuntersuchung bezeichnet werden. Das Mitteln der mehreren ersten Röntgenbilder kann ebenfalls als Teil der ersten Phase aufgefasst werden.

Das Bereitstellen des ersten Folgebildes und das Aufnehmen des zweiten Röntgenbildes kann als Teil einer zweiten Phase der Röntgenuntersuchung aufgefasst werden. In dieser zweiten Phase der Röntgenuntersuchung soll insbesondere eine Bewegung an der Körperregion, insbesondere die Bewegung eines medizinischen Objekts (beispielsweise Guidewire oder Katheter) oder eines Kontrastmittels, im Rahmen der Röntgenuntersuchung untersucht werden. Die zweite Phase findet zeitlich nach der ersten Phase statt, insbesondere direkt im Anschluss an die erste Phase. Das oben genannte zumindest eine erste Röntgenbild wird vorliegend so bezeichnet, da es in der ersten Phase aufgenommen wird, analog werden zweite Röntgenbilder so bezeichnet, da sie während der zweiten Phase aufgenommen werden. Das erste Folgebild kann ein weiteres zweites Röntgenbild sein, welches im Zuge des Bereitstellens aufgenommen wird. Alternativ kann das erste Folgebild aus mehreren zweiten Röntgenbildern gemittelt werden. Dieses Mitteln kann als Teil des Bereitstellens aufgefasst werden. Insbesondere wird das erste Folgebild durch Mitteln mehrerer zweiter Röntgenbilder erzeugt und bereitgestellt. Die hierfür herangezogenen mehreren zweiten Röntgenbilder repräsentieren die Körperregion vorteilhafter Weise alle zu einem früheren Aufnahmezeitpunkt als das zweite Folgebild. Mit anderen Worten werden die mehreren zweiten Röntgenbilder zum Bereitstellen des ersten Folgebildes zeitlich alle vor dem zweiten Röntgenbild aufgenommen. Wird nur ein einzelnes weiteres zweites Röntgenbild als das erste Folgebild bereitgestellt, so wird dieses vor dem zweiten Röntgenbild aufgenommen.

Der Aufnahmezeitpunkt des ersten Folgebildes kann dem Aufnahmezeitpunkt des einzelnen weiteren zweiten Röntgenbildes, welches als das erste Folgebild bereitgestellt wird, entsprechen. Wird das erste Folgebild aus mehreren zweiten Röntgenbildern bereitgestellt, so kann der Aufnahmezeitpunkt des ersten Folgebildes einem gemittelten Aufnahmezeitpunkt der mehreren zweiten Röntgenbilder oder einem Aufnahmezeitpunkt des aktuellsten der mehreren Röntgenbilder entsprechen. Der Aufnahmezeitpunkt des zweiten Röntgenbildes entspricht selbstredend dessen Aufnahmezeitpunkt.

Für das Bestimmen des Abweichungsmaßes können jeweilige Intensitäten des ersten Folgebildes und des zweiten Röntgenbildes ausgewertet werden. Insbesondere handelt es sich bei dem ersten Folgebild und dem zweiten Röntgenbild um Graustufenbilder, deren Graustufen jeweilige Intensitäten repräsentieren. Allgemein kann die Intensität einer durch einen Detektor des medizinischen Röntgengerätes erfassten Strahlungsintensität durch die Graustufen repräsentiert werden. Das vorliegende Abweichungsmaß kann dabei auf besonders einfache Weise durch Vergleichen jeweiliger Grauwerte des ersten Folgebildes und des zweiten Röntgenbildes bestimmt werden. Beispielsweise wird das Abweichungsmaß durch Subtraktion der jeweiligen Grauwerte des ersten und des zweiten Röntgenbildes bestimmt. Zusätzlich kann zum Bestimmen des Abweichungsmaßes ein Betrag eines Ergebnisses der Subtraktion bestimmt werden. Mit anderen Worten kann das Abweichungsmaß aus dem Betrag einer Differenz der jeweiligen Grauwerte des ersten und des zweiten Röntgenbildes gebildet werden.

Die vorbestimmte Vorschrift zum Bestimmen der Mittelungsgröße kann beispielsweise eine Zuordnungstabelle umfassen. Die Zuordnungstabelle kann jedem möglichen Wert des Abweichungsmaßes einen vorgegebenen Wert für die Mittelungsgröße zuweisen. Alternativ oder zusätzlich kann die vorbestimmte Vorschrift eine Verteilungsfunktion aufweisen. Mittels der Verteilungsfunktion ist ein mathematischer Zusammenhang zwischen dem Abweichungsmaß und der Mittelungsgröße bereitgestellt. Mittels der Verteilungsfunktion kann die Mittelungsgröße abhängig von dem Abweichungsmaß bestimmt werden.

Anschließend werden das erste Folgebild und das zweite Röntgenbild abhängig von der Mittelungsgröße gemischt, um das zweite Folgebild zu erzeugen. Das zweite Folgebild kann somit durch Mittelung des ersten Folgebildes und des zweiten Röntgenbildes erzeugt werden. Ob und zu welchem Grad eine Mittelung stattfindet, wird durch die Mittelungsgröße vorgegeben. Beispielsweise kann durch die Mittelungsgröße vorgegeben werden, dass das zweite Folgebild ausschließlich aus dem zweiten Röntgenbild erzeugt wird. In diesem Fall findet keine Mittelung statt. Alternativ kann durch die Mittelungsgröße vorgegeben werden, dass das zweite Folgebild aus dem zweiten Röntgenbild und dem ersten Folgebild erzeugt wird, wobei in diesem Fall ein Grad der Mittelung durch die Mittelungsgröße vorgegeben ist. Das Mitteln des ersten Folgebildes und des zweiten Röntgenbildes beim Erzeugen des zweiten Folgebildes betrifft dabei insbesondere jeweiligen Intensitätswerte der jeweils erfassten Strahlungsintensität, also beispielsweise die jeweiligen Grauwerte, des ersten Folgebildes und des zweiten Röntgenbildes. Das Mischen kann durch Mitteln der Intensitätswerte durchgeführt werden. Dabei kann die Mittelungsgröße als Gewichtungsfaktor bei dem Mitteln der Intensitätswerte aufgefasst werden. Mit anderen Worten werden beim Erzeugen des zweiten Folgebildes die jeweiligen Intensitätswerte beziehungsweise die jeweiligen Graustufen gemittelt. Die Mittelungsgröße kann hierbei ein Gewichtungsverhältnis angeben. Das zweite Folgebild kann somit als nachbearbeitetes Röntgenbild der zweiten Phase aufgefasst werden.

Beim Bilden des Gesamtbildes wird dieses aus dem Maskenbild und dem zweiten Folgebild zusammengesetzt. Beispielsweise knnen das Maskenbild und das zweite Folgebild zu dem Gesamtbild überlagert werden. Alternativ wird das Maskenbild von dem zweiten Folgebild subtrahiert. Eine Bildqualität des Gesamtbildes kann besonders hoch ausfallen, da einerseits eine Mittelung des Maskenbildes als auch eine Mittelung des zweiten Folgebildes erreicht werden kann. Auf diese Weise kann das jeweilige Bildrauschen sowohl des Maskenbildes als auch des zweiten Folgebildes zunächst jeweils eigenständig reduziert werden, bevor sich das jeweilige Bildrauschen des Maskenbildes und des zweiten Folgebildes in dem Gesamtbild addieren. Durch das Abweichungsmaß und die davon abhängige Mittelungsgröße ist sichergestellt, dass das Mitteln des ersten Folgebildes und des zweiten Röntgenbildes nur dann stattfindet, wenn eine Abweichung zwischen dem ersten Folgebild und dem zweiten Röntgenbild ein vorbestimmtes Maß nicht überschreitet. Auf diese Weise kann einem Kontrastverlust durch das Mitteln entgegengewirkt werden. Die genannte Abweichung zwischen dem ersten Folgebild und dem zweiten Röntgenbild kann insbesondere aus einer Bewegung an der Körperregion resultieren.

Gemäß einer Weiterbildung ist vorgesehen, dass bei dem Erzeugen des zweiten Folgebildes jeweilige Intensitätswerte von korrespondierenden Pixeln des ersten Folgebildes und des zweiten Röntgenbildes abhängig von der Mittelungsgröße gemischt werden. Die Intensitätswerte können jeweils durch Graustufen beziehungsweise Grauwerte des jeweiligen Folgebildes repräsentiert werden. Bei den Intensitätswerten handelt es sich um eine jeweilige detektierte Strahlungsintensität an dem Detektor des Röntgengeräts. Korrespondierende Pixel des ersten Folgebildes und des zweiten Röntgenbildes sind insbesondere Pixel gleicher Koordinaten. Mit anderen Worten werden die Intensitätswerte der jeweiligen Pixel des ersten Folgebildes und des zweiten Röntgenbildes mit jeweils gleichen Koordinaten gemischt beziehungsweise gemittelt. Auf diese Weise kann das Bildrauschen der einzelnen Pixel zumindest teilweise ausgemittelt werden.

Gemäß einer Weiterbildung ist vorgesehen, dass durch das Abweichungsmaß eine Bewegung an der Körperregion, welche in einem Zeitraum zwischen einem Aufnahmezeitpunkt des ersten Folgebildes und einem Aufnahmezeitpunkt des zweiten Röntgenbildes stattfindet, charakterisiert ist. Insbesondere handelt es sich bei dieser Bewegung an der Körperregion um die oben genannte Bewegung des medizinischen Objekts beziehungsweise des Kontrastmittels. Umso stärker die Bewegung ist, desto größer fällt das Abweichungsmaß aus. Mit anderen Worten kann das Abweichungsmaß ein Maß für eine Intensität der Bewegung sein. Es kann ein umso größerer Wert für das Abweichungsmaß bestimmt werden, je stärker die Bewegung an der Körperregion ist. Dadurch, dass die Bewegung an der Körperregion mittels des Abweichungsmaßes charakterisiert wird, kann eine Mittelung des ersten Folgebildes und des zweiten Röntgenbildes beim Erzeugen des zweiten Folgebildes bewegungsabhängig reduziert werden.

Gemäß einer Weiterbildung ist vorgesehen, dass durch die Mittelungsgröße festgelegt wird, zu welchem Anteil das zweite Folgebild dem zweiten Röntgenbild entspricht. Insbesondere wird durch die feste Mittelungsgröße festgelegt, zu welchem Anteil die Intensitätswerte beziehungsweise die Grauwerte des zweiten Folgebildes dem zweiten Röntgenbild entsprechen. Beispielsweise kann durch die Mittelungsgröße festgelegt werden, dass das zweite Folgebild vollständig dem zweiten Röntgenbild entspricht. In diesem Fall findet keine Mittelung statt. Alternativ kann durch die Mittelungsgröße festgelegt werden, dass eine Mittelung stattfindet und das zweite Folgebild zu einem Anteil, der durch die Mittelungsgröße festgelegt wird, dem zweiten Röntgenbild entspricht. Alternativ oder zusätzlich kann durch die Mittelungsgröße festgelegt werden, zu welchem Anteil das erste Folgebild dem zweiten Röntgenbild beim Erzeugen des zweiten Folgebildes beigemischt wird. Durch das Mischen beziehungsweise Mitteln entsprechend der Mittelungsgröße kann dieses besonders gut beeinflusst und angepasst werden.

Gemäß einer Weiterbildung ist vorgesehen, dass durch die Mittelungsgröße festgelegt ist, zu welchem Anteil eine Pixelintensität eines Pixels des zweiten Folgebildes einer Pixelintensität eines korrespondierenden Pixels des zweiten Röntgenbildes entspricht. Korrespondierende Pixel des zweiten Folgebildes, des ersten Folgebildes und des zweiten Röntgenbildes sind insbesondere jeweilige Pixel mit denselben Koordinaten. Alternativ oder zusätzlich kann durch die Mittelungsgröße festgelegt werden, zu welchem Anteil die Pixelintensität eines Pixels des zweiten Folgebildes einer Pixelintensität eines korrespondierenden Pixels des ersten Folgebildes entspricht. Beispielsweise entspricht die Pixelintensität eines Pixels des zweiten Folgebildes zu einem durch die Mittelungsgröße festgelegten Anteil der Pixelintensität des korrespondierenden Pixels des zweiten Röntgenbildes und zu einem weiteren durch die Mittelungsgröße festgelegten Anteil der Pixelintensität des korrespondierenden Pixels des ersten Folgebildes. Hierbei können der Anteil und der weitere Anteil zusammen eins ergeben.

Gemäß einer Weiterbildung ist vorgesehen, dass die Schritte c) bis e) für unterschiedliche Bereiche, insbesondere einzelne Pixel, des zweiten Folgebildes jeweils separat durchgeführt werden. Mit anderen Worten wird für die jeweiligen Bereiche, insbesondere die einzelnen Pixel, ein jeweiliges Abweichungsmaß und eine jeweilige Mittelungsgröße bestimmt. Das Erzeugen des zweiten Folgebildes aus dem zweiten Röntgenbild oder dem ersten Folgebild und dem zweiten Röntgenbild erfolgt dann bereichsweise für die unterschiedlichen Bereiche, insbesondere der einzelnen Pixel, des zweiten Folgebildes jeweils separat. Insbesondere wird mittels des Abweichungsmaßes die Bewegung in jedem der unterschiedlichen Bereiche, insbesondere einzelnen Pixel, jeweils separat charakterisiert und basierend darauf eine Bildinformation in den unterschiedlichen Bereichen des zweiten Folgebildes jeweils separat aus dem ersten Folgebild und dem zweiten Röntgenbild gemischt beziehungsweise gemittelt. Auf diese Weise kann die Bildqualität weiter verbessert werden.

Gemäß einer Weiterbildung ist vorgesehen, dass als das erste Folgebild ein solches bereitgestellt wird, welches zuvor analog zu den Schritten b) bis e) aus einem weiteren ersten Folgebild und einem weiteren zweiten Röntgenbild gebildet wurde. Mit anderen Worten kann das erste Folgebild bereits selbst durch Mischen mehrerer Bilder, nämlich dem weiteren ersten Folgebild und dem weiteren zweiten Röntgenbild, gebildet worden sein. Vorteilhafter Weise handelt es sich somit um ein iteratives Verfahren, bei welchem ein neues Folgebild stets entweder ausschließlich aus einem neuen zweiten Röntgenbild oder aus dem neuen zweiten Röntgenbild und einem vorherigen Folgebild gebildet wird. Dabei wird durch eine jeweilige Mittelungsgröße, die wiederum abhängig ist von einem jeweiligen Abweichungsmaß, bestimmt, um einen Anteil vorzugeben, zu welchem das vorherige Folgebild dem neuen zweiten Röntgenbild zum Bilden des neuen Folgebildes beigemischt wird. Beispielsweise kann ein Wertebereich der Mittelungsgröße vorgeben, dass das neue zweite Röntgenbild dem neuen Folgebild zu einem Prozentsatz von 20 % bis 100 % beigemischt wird. Auf diese Weise kann sich die Mittelung iterativ über mehrere Folgebilder hinwegziehen. Dabei wird ein Beitrag der mehreren Folgebilder zu dem aktuellen Folgebild umso größer, je neuer das jeweilige Bild ist.

Gemäß einer Weiterbildung ist vorgesehen, dass das Bestimmen der Mittelungsgröße anhand der vorbestimmten Vorschrift mittels einer Verteilungsfunktion, die eine Funktion des Abweichungsmaßes ist, erfolgt, wobei die Verteilungsfunktion abhängig von einem Betriebsparameter des Röntgengerätes bereitgestellt wird. Bei dem Betriebsparameter kann es sich um einen Beschleunigungsspannung, eine Dosis, eine Frequenz oder einen beliebigen anderen Parameter des Röntgengerätes handeln. Selbstverständlich kann die Verteilungsfunktion abhängig von mehreren Betriebsparametern des Röntgengerätes bereitgestellt werden. Beispielsweise wird die Verteilungsfunktion abhängig von dem Betriebsparameter aus einer Vielzahl von Verteilungsfunktionen, die in dem Röntgengerät gespeichert sind, ausgewählt. Alternativ kann die Verteilungsfunktionen basierend auf dem Betriebsparameter gebildet werden. Dabei können einer oder mehrere Punkte der Verteilungsfunktion aus dem Betriebsparameter berechnet werden und anschließend ein universeller Verlauf der Verteilungsfunktion, der insbesondere vorgegeben ist, an den berechneten beziehungsweise an die berechneten Punkte gefittet beziehungsweise angepasst werden. Auf diese Weise kann das Bestimmen des Abweichungsmaßes dahingehend verbessert werden, dass die Bewegung zielgerichteter charakterisiert werden kann.

Gemäß einer Weiterbildung ist vorgesehen, dass eine Rauschamplitude in dem ersten Folgebild und/oder dem zweiten Röntgenbild bestimmt wird und ein Schwellwert für die Verteilungsfunktion abhängig von der Rauschamplitude festgelegt wird. Insbesondere kann die Rauschamplitude als Betriebsparameter des Röntgengerätes aufgefasst werden. Durch den Schwellwert kann charakterisiert werden, ob eine im Rahmen des Abweichungsmaßes bestimmte Abweichung auf ein Bildrauschen oder eine Bewegung zurückzuführen ist. Beispielhaft wird für Werte des Abweichungsmaßes, die kleiner sind als der Schwellwert, die Mittelungsgröße derart bestimmt, dass ein größeres Maß an Mittelung der beim Erzeugen des zweiten Folgebild angewendet wird, als für Werte des Abweichungsmaßes, die größer sind als der Schwellwert. Auf diese Weise ist gewährleistet, dass das Bildrauschen verringert wird, aber im Falle einer Bewegung die Mittelung verringert oder ausgesetzt wird.

Gemäß einer Weiterbildung ist vorgesehen, dass bei dem Bilden des Gesamtbildes das Maskenbild und das zweite Folgebild voneinander subtrahiert werden. Mit anderen Worten wird das Gesamtbild durch subtrahieren des Maskenbildes von dem zweiten Folgebild erzeugt. Auf diese Weise kann die Bewegung, die insbesondere mittels der Röntgenuntersuchung untersucht werden soll, durch Subtraktion des statischen Bildinhalts besonders gut sichtbar gemacht werden.

Gemäß einer Weiterbildung ist vorgesehen, dass beim Aufnehmen des zumindest einen ersten Röntgenbildes mehrere erste Röntgenbilder aufgenommen werden und zum Erzeugen des Maskenbildes gemäß einer Rekursionsformel gemittelt werden. Mit anderen Worten erfolgt das Mitteln der mehreren ersten Röntgenbild nicht durch addieren der mehreren Röntgenbild und anschließendes Teilen durch die Anzahl, sondern rekursiv durch sukzessives Mitteln von Bild zu Bild. Dadurch kann eine Bildqualität bereits während der Erstellung des Maskenbildes verbessert werden.

Gemäß einer Weiterbildung ist vorgesehen, dass beim Erzeugen des Maskenbildes dieselbe Formel zum Mischen der mehreren ersten Röntgenbilder genutzt wird, wie bei dem Erzeugen des zweiten Folgebildes gemäß Schritt e) zum Mischen des ersten Folgebildes und des zweiten Röntgenbild es. Mit anderen Worten werden die mehreren ersten Röntgenbilder zum Erzeugen des Maskenbildes mittels derselben Formel gemischt beziehungsweise gemittelt, wie das erste Folgebild und das zweite Röntgenbild zum Erzeugen des zweiten Folgebildes. Auf diese Weise kann ein Umschalten zwischen der ersten Phase und der zweiten Phase besonders vorteilhaft durchgeführt werden, da in beiden Phasen dieselbe Formel genutzt wird.

Gemäß einer Weiterbildung ist vorgesehen, dass eine Positionsveränderung eines medizinischen Objekts zwischen dem ersten Folgebild und dem zweiten Röntgenbild bestimmt wird und jeweilige Objektbereiche des ersten Folgebildes und/oder des zweiten Röntgenbildes unter Anwendung einer Bewegungskompensation gemischt werden, wobei in den jeweiligen Objektbereichen das medizinische Objekt in dem ersten Folgebild und dem zweiten Röntgenbild erfasst wird. Bei dem medizinischen Objekt kann es sich beispielsweise um einen Draht (so genanntes Guidewire) oder einen Katheter handeln. Hierfür können zunächst die jeweiligen Objektbereiche in dem ersten Folgebild und dem zweiten Röntgenbild bestimmt werden. Die Positionsveränderung des medizinischen Objekts kann beispielsweise durch Vergleichen der Koordinaten der jeweiligen Objektbereiche bestimmt werden. Durch die Bewegungskompensation kann beispielsweise der Objektbereich des ersten Folgebildes derart verschoben und/oder rotiert werden, dass ein Maximum an Überlappung mit dem Objektbereich des zweiten Folgebildes erreicht wird. Dieses Maximum an Überlappung kann beispielsweise durch die Methode der kleinsten Quadrate bestimmt werden. Durch dieses kann ein Gesamtmaß an Abweichung zwischen den jeweiligen Objektbereichen definiert werden. Dieses Gesamtmaß an Abweichung kann im oben genannten Beispiel durch Verschieben des Objektbereichs des ersten Folgebildes verringert werden, bis ein (insbesondere absolutes) Minimum des Gesamtmaßes an Abweichung erreicht ist. Anschließend können die Objektbereiche gemischt werden. Dabei wird in einem weiteren Objektbereich das zweite Folgebild durch Mischen beziehungsweise Mitteln des Objektbereichs des ersten Folgebildes und des Objektbereichs des zweiten Röntgenbildes erzeugt. Der weitere Objektbereich des zweiten Folgebildes kann dieselben Koordinaten aufweisen, wie der Objektbereich des zweiten Röntgenbildes. Hierdurch ergibt sich ein verbesserter Kontrast in einem Bereich um das medizinische Objekt. Auf diese kann eine Bewegung des medizinischen Objekts mit verbesserter Bildqualität dargestellt werden.

Ein zweiter Aspekt der Erfindung betrifft ein medizinisches Röntgengerät zum Durchführen einer Röntgenuntersuchung, mit den Merkmalen des Anspruchs 13.

Insbesondere ist das medizinische Röntgengerät dazu eingerichtet, ein Verfahren, welches zuvor beschrieben wurde, durchzuführen. Merkmale des Verfahrens sowie Weiterbildungn des Verfahrens gelten somit analog auch für das medizinische Röntgengerät und bilden dieses auf analoge Weise weiter.

Die Erfindung wird nun anhand mehrerer Zeichnungen näher erläutert.

Dabei zeigen:

FIG 1     nach Art eines Blockschaltbilds ein medizinisches Röntgengerät;

FIG 2     einen schematischen Überblick über die Bildbearbeitung durch das medizinische Röntgengerät; und

FIG 3     eine beispielhafte Verteilungsfunktion für eine Mittelungsgröße.

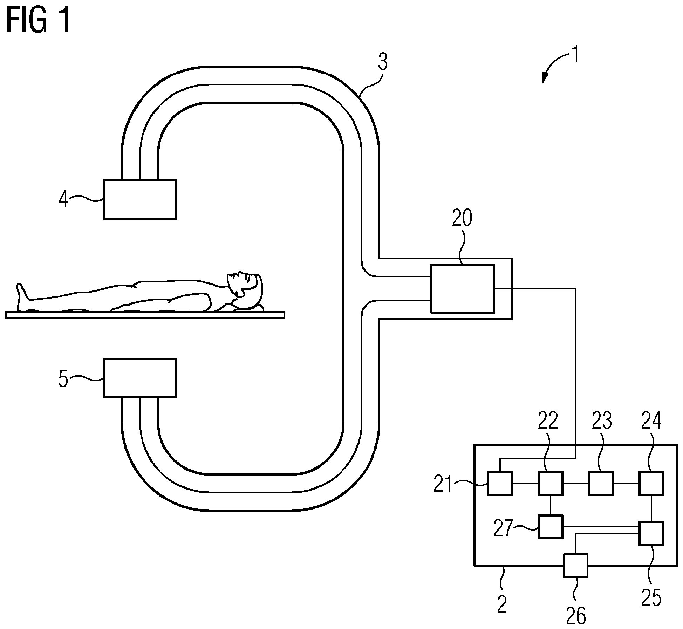

FIG 1 zeigt ein medizinisches Röntgengerät 1, insbesondere ein so genanntes C-Bogen-Röntgengerät. Das Röntgengerät 1 weist eine Röntgeneinrichtung 3 sowie eine Recheneinrichtung 2 auf. Die Röntgeneinrichtung 3 weist vorliegend eine Röntgenquelle 4 und einen Detektor 5, insbesondere Röntgendetektor, auf. Zudem umfasst die Röntgeneinrichtung 3 eine Aufnahmeeinheit 20 zum Aufnehmen von Röntgenbildern. Die Recheneinrichtung 2 weist vorlegend eine Bereitstellungseinheit 21, eine erste Bestimmungseinheit 22, eine zweite Bestimmungseinheit 23, eine Erzeugungseinheit 24, eine Zusammenfassungseinheit 25 und eine Ausgabeeinheit 26 auf. Die Ausgabeeinheit 26 ist dazu ausgebildet, eine Bildfolge an bearbeiteten Röntgenbildern auszugeben.

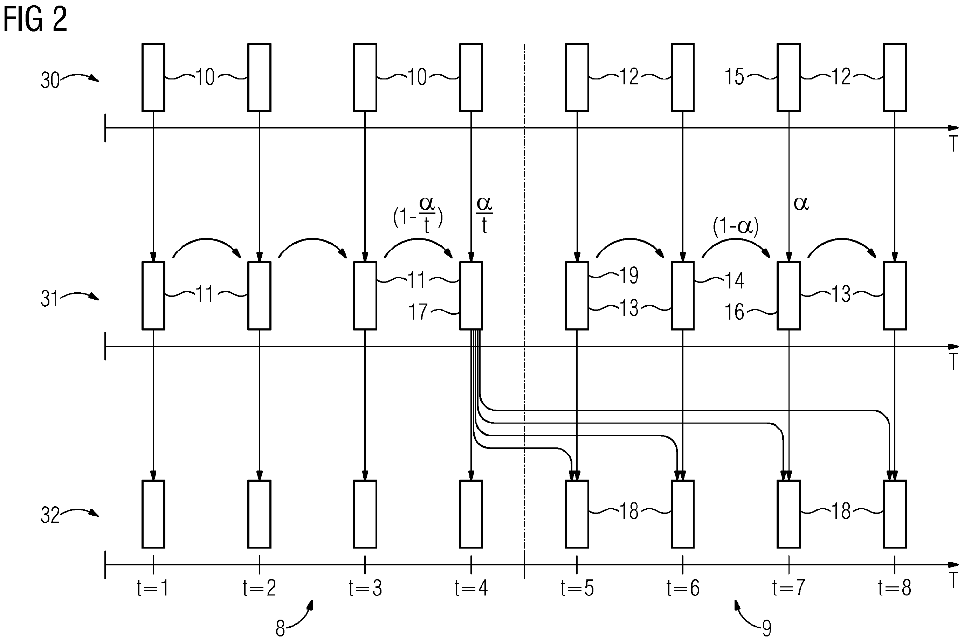

An FIG 2 soll nun ein Verfahren zum Betreiben des medizinischen Röntgengerätes 1 genauer erläutert werden. Hierzu zeigt FIG 2 in einer Übersicht eine Vielzahl an Röntgenbildern in unterschiedlichen Verarbeitungsschritten auf einer Zeitachse T. das Verfahren weist eine erste Phase 8 und eine zweite Phase 9 auf. Gemäß der Zeitachse T findet die erste Phase 8 vor der zweiten Phase 9 statt. Während der ersten Phase werden mehrere erste Röntgenbilder 10 durch das Röntgengerät 1 aufgenommen. Während der zweiten Phase 9 werden mehrere zweite Röntgenbilder 12 durch das medizinische Röntgengerät 1 aufgenommen. In der zweiten Phase 9 kann durch das medizinische Röntgengerät 1 eine Bewegung an der Körperstelle erfasst werden. Diese Bewegung kann beispielsweise durch die Zugabe eines Kontrastmittels in eine Körperflüssigkeit, welche die Körperregion durchströmt, gegeben sein. Alternativ kann die Bewegung eines medizinischen Objekts, beispielsweise eines Drahts (so genannte Guidewire) oder eines Katheters, durch das medizinische Röntgengerät 1 erfasst werden. Das Aufnehmen der ersten Röntgenbilder 10 und zweiten Röntgenbilder 12 kann als eine erste Verarbeitungsebene 30 aufgefasst werden. In der ersten Verarbeitungsebene 30 werden die ersten Röntgenbilder 10 sowie die zweiten Röntgenbilder 12 jeweils einzeln aufgenommen und optional jeweils einzeln weiter verarbeitet werden. Die ersten Röntgenbilder 10 sowie die zweiten Röntgenbilder 12 werden durch die Aufnahmeeinheit 20 aufgenommen.

Um die Bildqualität zu erhöhen, ist in einer zweiten Verarbeitungsebene 31 ein jeweiliges Mischen beziehungsweise Mitteln der ersten Röntgenbilder 10 und der zweiten Röntgenbilder 12 vorgesehen. Durch dieses Mitteln beziehungsweise Mischen kann ein Bildrauschen in den Bildern verringert werden. Hierzu kann entweder bei gleicher Dosis ein Signal-zu-Rausch-Abstand verbessert werden oder bei gleichem Signal-zu-Rausch-Abstand die Dosis verringert werden. Selbstverständlich können beide Möglichkeiten auch gemischt werden, das heißt die Dosis wird verringert und gleichzeitig der Signal-zu-Rausch-Abstand erhöht.

Die Röntgenbilder 10 der ersten Phase 8 werden vorliegend iterativ zu jeweiligen Maskenbildern 11 zusammengefasst. Dabei wird das Maskenbild 11 mit jedem neu hinzugekommenen ersten Röntgenbild 10 aktualisiert beziehungsweise neu gemittelt. Dies wird vorliegend mit der folgenden Formel 1 durchgeführt:

$$y_t^* = \frac{\alpha}{t} \cdot y_t + (1 - \frac{\alpha}{t}) \cdot y_{t-1}^*$$

Hierbei bezeichnet t die jeweilige Nummer eines Bildes auf der Zeitachse T. Mit anderen Worten kann t als Nummer des jeweiligen Frames (Bild einer Bildserie) bezeichnet werden. $\alpha$ bezeichnet eine Mittelungsgröße. $\alpha$ kann in der ersten Phase 8 insbesondere auf den Wert 1 gesetzt werden, da die Mittelungsgröße $\alpha$ zur bewegungsabhängigen Mittelung vorgesehen ist (siehe zweite Phase 9). Mit anderen Worten berechnet sich das erste Maskenbild 11 (t=1) nach folgender Formel:

$$y_t^* = 1 \cdot y_1 + (1 - 1) y_0^*$$

Das zweite Maskenbild 11 (t=2) berechnet sich gemäß folgender Formel:

$$y_2^* = \frac{1}{2} \cdot y_2 + (1 - \frac{1}{2}) y_1^*$$

Das dritte Maskenbild 11 (t=3) berechnet sich nach folgender Formel:

$$y_3^* = \frac{1}{3} \cdot y_3 + (1 - \frac{1}{3}) y_2^*$$

Dabei bezeichnet $y_t^*$ jeweils eines der Maskenbilder 11 mit der Nummer t. Mit anderen Worten bezeichnet $y_1^*$ das Maskenbild 11 mit der Nummer t=1, $y_2^*$ das Maskenbild 11 mit der Nummer t=2 und $y_3^*$

das Maskenbild 11 mit der Nummer t=3. Dementsprechend bezeichnet $y_{t-1}^*$ dasjenige der Maskenbilder 12 dessen Nummer t um 1 geringer ist, als die desjenigen Maskenbildes 12 mit der Nummer t. $y_0^*$ würde ein vorheriges Maskenbild bezeichnen, welches vorliegend nicht existiert, jedoch in der Formel zum Berechnen des Maskenbildes 11 für t=1 benötigt wird, da 1-1=0. $y_1$ bezeichnet das erste Röntgenbild 10 mit der Nummer t=1, $y_2$ bezeichnet das erste Röntgenbild 10 mit der Nummer t=2, $y_3$ bezeichnet das erste Röntgenbild 10 mit der Nummer t=3 und $y_t$ bezeichnet ein beliebiges erstes Röntgenbild 10 mit der Nummer t. Dementsprechend bezeichnet $y_{t-1}$ dasjenige der ersten Röntgenbilder 10 dessen Nummer t um 1 geringer ist als die desjenigen der Röntgenbilder 10 mit der Nummer t.

Im vorliegenden Beispiel werden vier erste Röntgenbilder 10 aufgenommen. Diese werden entsprechend der Formel 1 rekursiv gemittelt. Die Formel 1 entspricht einer arithmetischen Mittelung, wenn gilt $\alpha$=1. Im Gegensatz zu einer üblichen arithmetischen Mittelung erfolgt die Mittelung hierbei jedoch rekursiv, das bedeutet, es wird bei Hinzukommen eines neuen ersten Röntgenbildes 10 nicht über alle vorherigen Röntgenbilder 10 neu gemittelt, sondern nur das jeweilige vorherige der Röntgenbilder 10 für das Mitteln herangezogen. Das letzte Maskenbild 17 der vier Maskenbilder 11 wird am Ende der ersten Phase 8 für die zweite Phase 9 gespeichert.

In der zweiten Phase 9 werden Folgebilder 13 aus den zweiten Röntgenbildern 12 gebildet. Hierbei erfolgt eine Mittelung nach einer Formel, die analog zur Formel 1 zu verstehen ist. Im Gegensatz zu Formel 1 ist in der folgenden Formel 2 die Variabel t=1 gesetzt. Es handelt sich somit prinzipiell um dieselbe Formel wie beim Mischen beziehungsweise Mitteln der Maskenbilder 11. Die Formel 2 lautet wie folgt:

$$y_t^* = \alpha \cdot y_t + (1 - \alpha) \cdot y_{t-1}^*$$

In der Formel 3 bezeichnet $y_t^*$ dasjenige Folgebild 13 mit der Nummer t. $y_{t-1}^*$ bezeichnet dasjenige Folgebild 13 mit der Nummer t-1. $y_t$ bezeichnet dasjenige zweite Röntgenbild 12 mit der Nummer t. Die Anwendung dieser Formel wird im Folgenden beispielhaft anhand eines ersten Folgebildes 14, einem zweiten Röntgenbild 15 und einem zweiten Folgebild 16 genauer erläutert: Das erste Folgebild 14 trägt mit dem Faktor (1-$\alpha$) zu dem zweiten Folgebild 16 bei. Das zweite Röntgenbild 15 trägt mit dem Faktor $\alpha$ zu dem zweiten Folgebild 16 bei. Die Nummer t des zweiten Folgebildes 16 lautet im vorliegenden Beispiel t=7. Somit lautet die Formel zum Berechnen des zweiten Folgebildes 16 vorliegend wie folgt (Formel 3):

$$y_7^* = \alpha \cdot y_7 + (1 - \alpha) \cdot y_6^*$$

In der Formel 3 bezeichnet $y_7^*$ das zweite Folgebild 16. $y_6^*$ bezeichnet das erste Folgebild 14. $y_7$ bezeichnet das zweite Röntgenbild 15. Die Mittelungsgröße $\alpha$ ist abhängig von einer Abweichung zwischen dem ersten Folgebild 14 und dem zweiten Röntgenbild 15. Bei dem vorliegenden Röntgengerät ist die Erzeugungseinheit 24 (siehe FIG 1) zum Erzeugen des zweiten Folgebildes 16 aus dem ersten Folgebild 14 und dem zweiten Röntgenbild 15 oder aus dem zweiten Röntgenbild 15 ausgebildet.

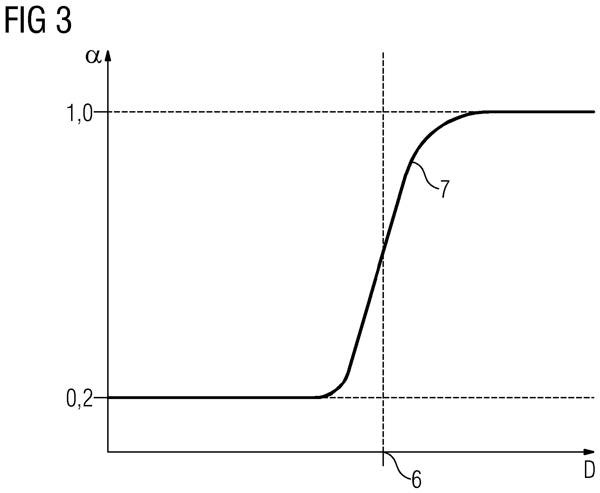

Zum Bestimmen der Mittelungsgröße $\alpha$ wird zunächst ein Abweichungsmaß D bestimmt. Vorliegend ist die erste Bestimmungseinheit 22 zum Bestimmen des Abweichungsmaßes ausgebildet. Zum Bestimmen des Abweichungsmaßes wird eine Abweichung zwischen dem ersten Folgebild 14 und dem zweiten Röntgenbild 15 bestimmt. Vorliegend wird das Abweichungsmaß für jeden Pixel des zweiten Röntgenbildes 15 beziehungsweise des ersten Folgebildes 14 einzeln bestimmt. Hierzu wird jeweils eine Differenz zwischen den zugrunde liegenden Intensitäten beziehungsweise Grauwerten korrespondierender Pixel des zweiten Röntgenbildes 15 und des ersten Folgebildes 14 gebildet. Aus diesen wird der Betrag der jeweiligen Differenz gebildet. Diese beiden Schritte (Bilden der Differenz, Bilden des Betrags) werden für alle Pixel des Folgebildes 14 und des zweiten Röntgenbildes 15 jeweils separat durchgeführt. Korrespondierende Pixel des ersten Folgebildes 14 und des zweiten Röntgenbildes 15 sind vorliegend Pixel, welche dieselben Koordinaten aufweisen. Die jeweiligen Beträge der jeweiligen Differenz bilden das jeweilige Abweichungsmaß D für die jeweiligen korrespondierenden Pixel. Anschließend wird für jeden Pixel des zweiten Röntgenbildes 15 beziehungsweise des ersten Folgebildes 14 eine jeweilige Mittelungsgröße $\alpha$ bestimmt. Vorliegend ist die zweite Bestimmungseinheit 23 des Röntgengerätes 1 zum Bestimmen der Mittelungsgröße $\alpha$ abhängig von dem Abweichungsmaß D nach einer vorbestimmten Vorschrift ausgebildet. FIG 3 zeigt eine Verteilungsfunktion 7, welche Teil der vorbestimmten Vorschrift zum Bestimmten der Mittelungsgröße $\alpha$ ist. Durch die Verteilungsfunktion 7 kann jedem Wert des Abweichungsmaßes D ein jeweiliger Wert für die Mittelungsgröße $\alpha$ zugeordnet werden. Vorliegend weist die Mittelungsgröße $\alpha$ einen Wertbereich von 0,2 bis 1 auf. Nimmt die Mittelungsgröße $\alpha$ den Wert 1 an, so wird das zweite Folgebild 16 ausschließlich aus dem zweiten Röntgenbild 15 gebildet. Mit sinkendem Wert der Mittelungsgröße $\alpha$ steigt der Anteil, zu welchem das zweite Folgebild 16 aus dem ersten Folgebild 14 gebildet wird.

Die Formel 2 beziehungsweise die Formel 3 wird vorliegend für jeden Pixel des zweiten Folgebildes 16 einzeln angewendet. Mit anderen Worten kann eine Intensität jedes Pixels des zweiten Folgebildes 16 mittels der Formel 2 beziehungsweise Formel 3 jeweils einzeln berechnet beziehungsweise bestimmt werden. Auf diese Weise werden Bildbereiche, in denen ein großes Maß an Veränderung zwischen dem ersten Folgebild 14 und dem zweiten Röntgenbild 15 vorliegt, gar nicht gemittelt oder weniger stark gemittelt als Bildbereiche, in denen ein geringes Maß an Veränderung zwischen dem ersten Folgebild 14 und dem zweiten Röntgenbild 15 vorliegt. Als hohes Maß an Veränderung gilt hierbei insbesondere ein Abweichungsmaß D welches größer ist als ein vorbestimmter Schwellwert 6 (siehe FIG 4). Als geringes Maß an Veränderung gilt insbesondere ein Abweichungsmaß D, welches kleiner ist als der vorbestimmte Schwellwert 6.

Um das Bestimmen der Mittelungsgröße $\alpha$ besonders gut an einen Betriebszustand des Röntgengerätes 1 anpassen zu können, ist vorliegend vorgesehen, dass die Verteilungsfunktion 7 abhängig von einem Betriebsparameter des Röntgengerätes 1 ausgewählt wird. Beispielsweise sind mehrere unterschiedliche Verteilungsfunktionen 7 für unterschiedliche Betriebszustände in dem Röntgengerät 1 gespeichert. Abhängig von einem aktuellen Betriebsparameter des Röntgengerätes 1 kann dann die für den momentanen Betriebszustand passende der Verteilungsfunktionen 7 ausgewählt werden. Beispiele für Betriebsparameter sind eine oder mehrere der folgenden: zu nutzen der Dosis, Beschleunigungsspannung, Röntgenfrequenz, zu untersuchende Körperregion oder physiologische Daten (Körpergröße, Körpergewicht oder Geschlecht einer zu untersuchenden Person). Die unterschiedlichen Verteilungsfunktionen 7 können insbesondere durch einen jeweiligen Schwellwert 6 charakterisiert sein. Der Schwellwert 6 entspricht insbesondere einem Wendepunkt der Verteilungsfunktion 7. Die Verteilungsfunktion 7 kann einer Treppenfunktion beziehungsweise Tetha-Funktion, auch Heavyside-Funktion genannt, nachempfunden sein. Hierbei ist die Verteilungsfunktion 7 jedoch um den Schwellwert 6 parallel zur x-Achse beziehungsweise vorliegend D-Achse, verschoben. Zudem ist die Verteilungsfunktion 7 verglichen mit der Tetha-Funktion vorliegend im Bereich um den Sprung, also im Bereich um den Schwellwert 6, abgerundet.

Dementsprechend ergibt sich eine zweite Möglichkeit, die Verteilungsfunktion 7 bereitzustellen. Beispielsweise sind in dem Röntgengerät 1 nicht mehrere unterschiedliche Verteilungsfunktionen 7 gespeichert, sondern mehrere Schwellwerte 6 und/oder Werte für die Abrundung im Bereich des Schwellwerts 6 für unterschiedliche Werte des Betriebsparameters. Anschließend kann die Verteilungsfunktion 7 ausgehend von der Tetha-Funktion unter Berücksichtigung des Schwellwerts 6 und/oder des Maßes für die Abrundung an dem Schwellwert 6 gebildet werden.

Vorliegend ist in FIG 2 eine dritte Verarbeitungsebene 32 gezeigt. In der dritten Verarbeitungsebene 32 ist gezeigt, welche Röntgenbilder durch die Ausgabeeinheit 26 für die Untersuchung ausgegeben werden. In der ersten Phase 8 kann jeweils das aktuellste Maskenbild 11, das Maskenbild 11, mit der aktuell höchsten Nummer t, ausgegeben werden. In der zweiten Phase 9 wird jeweils das aktuellste Folgebild 13, also das Folgebild 13 mit der größten Nummer t, subtrahiert und das letzte beziehungsweise aktuellste Maskenbild 17 ausgegeben. Mit anderen Worten wird aus jedem der Folgebild 13 ein Gesamtbild 18 durch Subtrahieren des letzten Maskenbildes 17 gebildet. Diese Gesamtbilder 18 werden gemäß der Zeitachse T nacheinander ausgegeben. Es handelt sich somit vorliegend um eine Subtraktionsbildgebung. Die Zusammenfassungseinheit 25 ist vorliegend zum Bilden der Gesamtbilder 18 durch Subtrahieren des letzten Maskenbildes 17 von dem jeweiligen Folgebild 13 ausgebildet.

Ein phasenerstes Folgebild 19, also das erste der Folgebilder 13 in der zweiten Phase 9, kann je nach Ausführungsform nicht gemittelt werden, das bedeutet, α wird beim Berechnen des phasenersten Folgebilds 19 auf den Wert 1 gesetzt, oder zum Mitteln des phasenersten Folgebildes 19 wird das letzte Maskenbild 17 herangezogen. In diesem Fall kann das Bildrauschen bereits in dem phasenersten Folgebild 19 und somit bereits im ersten Gesamtbild 18 der zweiten Phase 9 verbessert werden.

Vorzugsweise ist vorgesehen, dass der Schwellwert 6 durch das Auswerten von Rauschamplituden zumindest teilweise vorgegeben wird. Mit anderen Worten kann der Schwellwert 6 zumindest teilweise basierend auf einer Rauschamplitude in einem oder mehreren der Röntgenbilder 10, 12 bereitgestellt werden. Vorzugsweise ist der Schwellwert 6 stets größer als die berechnete Rauschamplitude. Auf diese Weise kann vermieden werden, dass ein Rauschen fälschlicherweise als Bewegung erkannt wird.

Das medizinische Röntgengerät 1 kann eine Bewegungskompensationseinheit 27 zum Bereitstellen einer Bewegungskompensation aufweisen, um die Darstellung der Bewegung eines medizinischen Objekts zu verbessern. Vorliegend ist vorgesehen, dass eine Positionsveränderung des medizinischen Objekts zwischen dem ersten Folgebild 14 und dem zweiten Röntgenbild 15 bestimmt wird und jeweilige Objektbereiche des ersten Folgebildes 14 und/oder des zweiten Röntgenbildes 15 unter Anwendung der Bewegungskompensation gemischt werden. In den jeweiligen Objektbereichen wird das medizinische Objekt in dem ersten Folgebild 14 beziehungsweise dem zweiten Röntgenbild 15 erfasst. Zunächst können die jeweiligen Objektbereiche in dem ersten Folgebild 14 und dem zweiten Röntgenbild 15 bestimmt werden. Die Positionsveränderung des medizinischen Objekts kann beispielsweise durch Vergleichen der Koordinaten der jeweiligen Objektbereiche bestimmt werden. Durch die Bewegungskompensation kann beispielsweise der Objektbereich des ersten Folgebildes 14 derart verschoben und/oder rotiert werden, dass ein Maximum an Überlappung mit dem Objektbereich des zweiten Folgebildes erreicht wird. Dieses Maximum an Überlappung wird im vorliegenden Beispiel durch die Methode der kleinsten Quadrate bestimmt. Durch diese wird ein Gesamtmaß an Abweichung zwischen den jeweiligen Objektbereichen definiert. Dieses Gesamtmaß an Abweichung wird vorliegend durch Verschieben des Objektbereichs des ersten Folgebildes 14 verringert werden, bis ein (insbesondere absolutes) Minimum des Gesamtmaßes an Abweichung erreicht ist. Anschließend können die Objektbereiche gemischt werden. Mit anderen Worten wird in einem weiteren Objektbereich das zweite Folgebild 16 durch Mischen beziehungsweise Mitteln des Objektbereichs des ersten Folgebildes 14 und des Objektbereichs des zweiten Röntgenbildes 15 erzeugt. Der weitere Objektbereich des zweiten Folgebildes 16 kann dieselben Koordinaten aufweisen, wie der Objektberiech des zweiten Röntgenbildes 15. Für das Mischen beziehungsweise Mitteln kann die oben genannte Formel 2 genutzt werden.

Insgesamt ist durch das Ausführungsbeispiel gezeigt, wie die Bildqualität im Vergleich zum Stand der Technik verbessert werden kann.

**Patentansprüche**

1. Verfahren zum Betreiben eines medizinischen Röntgengerätes (1) beim Durchführen einer Röntgenuntersuchung, mit den Verfahrensschritten:

   a) Aufnehmen mehrerer erster Röntgenbilder (10) einer Körperregion in einer ersten Phase (8) der Röntgenuntersuchung,
   wobei die mehreren ersten Röntgenbilder (10) gemäß einer Rekursionsformel zu einem Maskenbild (17) gemittelt werden,
   wobei das Maskenbild (17) mit jedem neu hinzugekommenen ersten Röntgenbild (10) aktualisiert wird,
   b) Bereitstellen eines ersten Folgebildes (14) und Aufnehmen eines zweiten Röntgenbildes (15) der Körperregion in einer zweiten Phase der Röntgenuntersuchung,
   wobei das zweite Röntgenbild (15) die Körperregion zu einem späteren Aufnahmezeitpunkt repräsentiert als das erste Folgebild (14),
   wobei bei erstmaliger Ausführung der Schritte b) bis e) als das erste Folgebild (14) das zuletzt aktualisierte Maskenbild (17) der ersten Phase (8) vorgegeben wird,
   c) Bestimmen eines Abweichungsmaßes (D), welches eine Abweichung zwischen dem ersten Folgebild (14) und dem zweiten Röntgenbild (15) betrifft,
   d) Bestimmen einer Mittelungsgröße (α) abhängig von dem Abweichungsmaß (D) nach einer vorbestimmten Vorschrift, wobei das Bestimmen der Mittelungsgröße ($\alpha$) anhand der vorbestimmten Vorschrift mittels einer Verteilungsfunktion (7), die eine Funktion des Abweichungsmaßes (D) ist, erfolgt, wobei die Verteilungsfunktion (7) abhängig von einem Betriebsparameter des Röntgengerätes (1) bereitgestellt wird,

e) Erzeugen eines zweiten Folgebildes (16) aus dem ersten Folgebild (14) zusammen mit dem zweiten Röntgenbild (15), wobei durch die Mittelungsgröße ($\alpha$) vorgegeben ist, zu welchen Anteilen das erste Folgebild (14) und das zweite Röntgenbild (15) hierbei gemischt werden,

f) Bilden eines Gesamtbildes (18) aus dem Maskenbild (17) und dem zweiten Folgebild (16).

2. Verfahren nach Anspruch 1, **dadurch gekennzeichnet, dass** bei dem Erzeugen des zweiten Folgebildes (16) jeweilige Intensitätswerte von korrespondierenden Pixeln des ersten Folgebildes (14) und des zweiten Röntgenbildes (15) abhängig von der Mittelungsgröße ($\alpha$) gemischt werden.

3. Verfahren nach einem der vorhergehenden Ansprüche, **dadurch gekennzeichnet, dass** durch das Abweichungsmaß (D) eine Bewegung an der Körperregion, welche in einem Zeitraum zwischen einem Aufnahmezeitpunkt des ersten Folgebildes (14) und einem Aufnahmezeitpunkt des zweiten Röntgenbildes (15) stattfindet, charakterisiert wird.

4. Verfahren nach einem der vorhergehenden Ansprüche, **dadurch gekennzeichnet, dass** durch die Mittelungsgröße ($\alpha$) festgelegt wird, zu welchem Anteil das zweite Folgebild (16) dem zweiten Röntgenbild (15) entspricht.

5. Verfahren nach einem der vorhergehenden Ansprüche, **dadurch gekennzeichnet, dass** durch die Mittelungsgröße ($\alpha$) festgelegt wird, zu welchem Anteil eine Pixelintensität eines Pixels des zweiten Folgebildes (16) einer Pixelintensität eines korrespondierenden Pixels des zweiten Röntgenbildes (15) entspricht.

6. Verfahren nach einem der vorhergehenden Ansprüche, **dadurch gekennzeichnet, dass** ein Betrag einer Differenz jeweiliger Pixelintensitäten korrespondierender Pixel des ersten Folgebildes (14) und des zweiten Röntgenbildes (15) als das Abweichungsmaß (D) bestimmt wird.

7. Verfahren nach einem der vorhergehenden Ansprüche, **dadurch gekennzeichnet, dass** die Schritte c) bis e) für unterschiedliche Bereiche, insbesondere einzelne Pixel, des zweiten Folgebildes (16) jeweils separat durchgeführt werden.

8. Verfahren nach einem der vorhergehenden Ansprüche, **dadurch gekennzeichnet, dass** als das erste Folgebild (14) ein solches bereitgestellt wird, welches zuvor analog zu den Schritten b) bis e) aus einem weiteren ersten Folgebild (13) und einem weiteren zweiten Röntgenbild (12) gebildet wurde.

9. Verfahren nach einem der vorhergehenden Ansprüche, **dadurch gekennzeichnet, dass** eine Rauschamplitude in dem ersten Folgebild (14) und/oder dem zweiten Röntgenbild (15) bestimmt wird und ein Schwellwert (6) für die Verteilungsfunktion (7) abhängig von der Rauschamplitude festgelegt wird.

10. Verfahren nach einem der vorhergehenden Ansprüche, **dadurch gekennzeichnet, dass** bei dem Bilden des Gesamtbildes (18) das Maskenbild (17) und das zweite Folgebild (16) voneinander subtrahiert werden.

11. Verfahren nach einem der vorhergehenden Ansprüche, **dadurch gekennzeichnet, dass** beim Aufnehmen des zumindest einen ersten Röntgenbildes (10) mehrere erste Röntgenbilder (10) aufgenommen werden und bei dem Erzeugen des Maskenbildes (11, 17) dieselbe Formel zum Mischen der mehreren ersten Röntgenbilder (10) genutzt wird, wie bei dem Erzeugen des zweiten Folgebildes (16) gemäß Schritt e) zum Mischen des ersten Folgebildes (14) und des zweiten Röntgenbildes (15).

12. Verfahren nach einem der vorhergehenden Ansprüche, **dadurch gekennzeichnet, dass** eine Positionsveränderung eines medizinischen Objekts zwischen dem ersten Folgebild (14) und dem zweiten Röntgenbild (15) bestimmt wird und jeweilige Objektbereiche des ersten Folgebildes (14) und/oder des zweiten Röntgenbildes (15) unter Anwendung einer Bewegungskompensation gemischt werden, wobei in den jeweiligen Objektbereichen das medizinische Objekt in dem ersten Folgebild (14) und dem zweiten Röntgenbild (15) erfasst wird.

13. Medizinisches Röntgengerät (1) zum Durchführen einer Röntgenuntersuchung, mit

    - einer Aufnahmeeinheit (20) ausgebildet:

       - zum Aufnehmen mehrerer erster Röntgenbilder (10) einer Körperregion in einer ersten Phase (8) der Röntgenuntersuchung,
       - zum Mitteln der mehreren ersten Röntgenbilder (10) gemäß einer Rekursionsformel zu einem Maskenbild (17),
       - zum Aktualisieren des Maskenbildes (17) mit jedem neu hinzugekommenen ersten Röntgenbild (10),
- zum Vorgeben des zuletzt aktualisierten Maskenbildes (14) der ersten Phase als erstes Folgebild (14), und
- zum Aufnehmen eines zweiten Röntgenbildes (15) der Körperregion in einer zweiten Phase der Röntgenuntersuchung;

- einer Bereitstellungseinheit (21) zum Bereitstellen des ersten Folgebildes (14) der Körperregion,
wobei das zweite Röntgenbild (15) die Körperregion zu einem späteren Aufnahmezeitpunkt repräsentiert als das erste Folgebild (14);
- einer ersten Bestimmungseinheit (22) zum Bestimmen eines Abweichungsmaßes (D), welches eine Abweichung zwischen dem ersten Folgebild (14) und dem zweiten Röntgenbild (15) betrifft,
- einer zweiten Bestimmungseinheit (23) zum Bestimmen einer Mittelungsgröße (α) abhängig von dem Abweichungsmaß (D) nach einer vorbestimmten Vorschrift;
- einer Erzeugungseinheit (24) zum Erzeugen eines zweiten Folgebildes (16) aus dem ersten Folgebild (14) zusammen mit dem zweiten Röntgenbild (15),
wobei durch die Mittelungsgröße (α) vorgegeben ist, zu welchen Anteilen das erste Folgebild (14) und das zweite Röntgenbild (15) hierbei durch die Erzeugungseinheit (24) zu mischen sind, und
- einer Zusammenfassungseinheit (25) zum Bilden eines Gesamtbildes (18) aus dem Maskenbild (17) und dem zweiten Folgebild (16).

## Claims

1. Method for operating a medical X-ray device (1) when performing an X-ray examination, with the method steps:

   a) recording a plurality of first X-ray images (10) of a body region in a first phase (8) of the X-ray examination,
   wherein the plurality of first X-ray images (10) is averaged according to a recurrence formula to generate a mask image (17),
   wherein the mask image (17) is updated with each newly accrued first X-ray image (10),
   b) providing a first subsequent image (14) and recording a second X-ray image (15) of the body region in a second phase of the X-ray examination,
   wherein the second X-ray image (15) represents the body region at a later recording time than the first subsequent image (14) ;
wherein when initially executing the steps b) to e) as the first subsequent image (14), the last updated mask image (17) of the first phase (8) is specified,
c) determining a degree of deviation (D) relating to a deviation between the first subsequent image (14) and the second X-ray image (15),
d) determining an averaging amount (α) in dependence on the degree of deviation (D) according to a predetermined rule, wherein the determination of the averaging amount (α) is performed using the predetermined rule by means of a distribution function (7), which is a function of the degree of deviation (D), wherein the distribution function (7) is provided in dependence on an operating parameter of the X-ray device (1),
e) generating a second subsequent image (16) from the first subsequent image (14) together with the second X-ray image (15), wherein the averaging amount (α) specifies the proportions in which the first subsequent image (14) and the second X-ray image (15) are mixed thereby,
f) forming an overall image (18) from the mask image (17) and the second subsequent image (16).

2. Method according to claim 1, **characterised in that** during the generation of the second subsequent image (16) respective intensity values of corresponding pixels of the first subsequent image (14) and the second X-ray image (15) are mixed in dependence on the averaging amount (α).

3. Method according to one of the preceding claims, **characterised in that** the degree of deviation (D) characterises a movement at the body region that occurs in a period between a recording time of the first subsequent image (14) and a recording time of the second X-ray image (15).

4. Method according to one of the preceding claims, **characterised in that** the averaging amount (α) defines the proportion in which the second subsequent image (16) corresponds to the second X-ray image (15).

5. Method according to one of the preceding claims, **characterised in that** the averaging amount (α) defines the proportion in which a pixel intensity of a pixel of the second subsequent image (16) corresponds to a pixel intensity of a corresponding pixel of the second X-ray image (15).

6. Method according to one of the preceding claims, **characterised in that** an amount of a difference between pixels of the first subsequent image (14) and the second X-ray image (15) corresponding to respective pixel intensities is determined as the degree of deviation (D).

7. Method according to one of the preceding claims, **characterised in that** steps c) to e) are carried out separately in each case for different regions, in particular individual pixels, of the second subsequent image (16).

8. Method according to one of the preceding claims, **characterised in that** the first subsequent image (14) provided is one which was formed previously similarly to steps b) to e) from a further first subsequent image (13) and a further second X-ray image (12).

9. Method according to one of the preceding claims, **characterised in that** a noise amplitude is determined in the first subsequent image (14) and/or the second X-ray image (15) and a threshold value (6) for the distribution function (7) is defined in dependence on the noise amplitude.

10. Method according to one of the preceding claims, **characterised in that** during the formation of the overall image (18), the mask image (17) and the second subsequent image (16) are subtracted from one another.

11. Method according to one of the preceding claims, **characterised in that** during the recording of the at least one first X-ray image (10), a plurality of first X-ray images (10) are recorded and during the generation of the mask image (11, 17), the same formula is used to mix the plurality of first X-ray images (10) as during the generation of the second subsequent image (16) according to step e) for mixing the first subsequent image (14) and the second X-ray image (15).

12. Method according to one of the preceding claims, **characterised in that** a change in position of a medical object between the first subsequent image (14) and the second X-ray image (15) is determined and respective object regions of the first subsequent image (14) and/or the second X-ray image (15) are mixed using motion compensation, wherein the medical object is detected in the first subsequent image (14) and the second X-ray image (15) in the respective object regions.

13. Medical X-ray device (1) for performing an X-ray examination, with

    - a recording unit (20) embodied:

        - for recording a plurality of first X-ray images (10) of a body region in a first phase (8) of the X-ray examination,
    - for averaging the plurality of first X-ray images (10) according to a recurrence formula to form a mask image (17),
    - for updating the mask image (17) with each newly accrued first X-ray image (10),
    - for predetermining the last updated mask image (14) of the phase as a first subsequent image (14), and
    - for recording a second X-ray image (15) of the body region in a second phase of the X-ray examination;

- a providing unit (21) for providing the first subsequent image (14) of the body region,
wherein the second X-ray image (15) represents the body region at a later recording time than the first subsequent image (14) ;
- a first determining unit (22) for determining a degree of deviation (D) relating to a deviation between the first subsequent image (14) and the second X-ray image (15),
- a second determining unit (23) for determining an averaging amount ($\alpha$) in dependence on the degree of deviation (D) according to a predetermined rule;
- a generating unit (24) for generating a second subsequent image (16) from the first subsequent image (14) together with the second X-ray image (15),
wherein the averaging amount ($\alpha$) specifies the proportions in which the first subsequent image (14) and the second X-ray image (15) are to be mixed thereby by the generating unit (24) and
- a combining unit (25) for forming an overall image (18) from the mask image (17) and the second subsequent image (16).

**Revendications**

1. Procédé pour faire fonctionner un appareil (1) médical à rayons X lorsque l'on effectue un examen aux rayons X, comprenant les stades de procédé :

    a) Enregistrement de plusieurs images (10) de rayons X d'une région du corps dans une première phase (8) de l'examen aux rayons X, dans lequel on fait une moyenne des plusieurs premières images (10) de rayons X en une image (17) de masque suivant une formule de récursion,
dans lequel on met à jour l'image (17) de masque à chaque première image (10) de rayons X arrivée nouvellement,
b) Mise à disposition d'une première image (14) successive et enregistrement d'une deuxième image (15) de rayons X de la région du corps dans une deuxième phase de l'examen aux rayons X,

dans lequel la deuxième image (15) de rayons X représente la région du corps à un instant d'enregistrement plus tardif que la première image (14) successive,

dans lequel, lorsque l'on exécute pour la première fois les stades b) à e), on prescrit comme première image (14) successive l'image (17) de masque mise à jour en dernier de la première phase (8),

c) Détermination d'une mesure (D) d'écart, qui concerne un écart entre la première image (14) successive et la deuxième image (15) de rayons X,

d) Détermination d'une grandeur (a) d'établissement d'une moyenne en fonction de la mesure (D) d'écart suivant une prescription déterminée à l'avance,

dans lequel la détermination de la grandeur ($\alpha$) d'établissement d'une moyenne s'effectue à l'aide de la prescription déterminée à l'avance au moyen d'une fonction (7) de répartition, qui est une fonction de la mesure (D) d'écart, la fonction (7) de répartition étant mise à disposition en fonction d'un paramètre de fonctionnement de l'appareil (1) à rayons X,

e) Production d'une deuxième image (16) successive à partir de la première image (14) successive ensemble avec la deuxième image (15) de rayons X, dans lequel il est prescrit par la grandeur ($\alpha$) d'établissement d'une moyenne les proportions en lesquelles la première image (14) successive et la deuxième image (15) de rayons X sont mélangées,

f) Formation d'une image (18) d'ensemble composée de l'image (17) de masque et de la deuxième image (16) successive.

2. Procédé suivant la revendication 1, **caractérisé en ce que**, lors de la production de la deuxième image (16) successive, on mélange des valeurs d'intensité respectives de pixels correspondants de la première image (14) successive et de la deuxième image (15) de rayons X en fonction de la grandeur ($\alpha$) d'établissement d'une moyenne.

3. Procédé suivant l'une des revendications précédentes, **caractérisé en ce que**, par la mesure (D) d'écart, on caractérise un déplacement sur la région du corps, qui a lieu dans un laps de temps entre un instant d'enregistrement de la première image (14) successive et un instant d'enregistrement de la deuxième image (15) de rayons X.

4. Procédé suivant l'une des revendications précédentes, **caractérisé en ce que**, par la grandeur ($\alpha$) d'établissement d'une moyenne, on fixe la proportion en laquelle la deuxième image (16) successive correspond à la deuxième image (15) de rayons X.

5. Procédé suivant l'une des revendications précédentes, **caractérisé en ce que**, par la grandeur ($\alpha$) d'établissement d'une moyenne, on fixe la proportion en laquelle une intensité d'un pixel de la deuxième image (16) successive correspond à une intensité d'un pixel correspondant de la deuxième image (15) de rayons X.

6. Procédé suivant l'une des revendications précédentes, **caractérisé en ce qu'**on détermine comme mesure (D) d'écart une valeur absolue d'une différence entre des intensités de pixels correspondants de la première image (14) successive et de la deuxième image (15) de rayons X.

7. Procédé suivant l'une des revendications précédentes, **caractérisé en ce que** l'on effectue respectivement séparément les stades c) à e) pour des parties différentes, notamment des pixels individuels, de la deuxième image (16) successive.

8. Procédé suivant l'une des revendications précédentes, **caractérisé en ce que** l'on met à disposition comme première image (14) successive une image telle qu'a été formée auparavant d'une manière analogue aux stades b) à e) à partir d'une autre première image (13) successive et d'une autre deuxième image (12) de rayons X.

9. Procédé suivant l'une des revendications précédentes, **caractérisé en ce que** l'on détermine une amplitude de bruit dans la première image (14) successive et/ou dans la deuxième image (15) de rayons X et on fixe une valeur (6) de seuil pour la fonction (7) de répartition en fonction de l'amplitude de bruit.

10. Procédé suivant l'une des revendications précédentes, **caractérisé en ce que**, lors de la formation de l'image (18) d'ensemble, on soustrait, l'une de l'autre, l'image (17) de masque et la deuxième image (16) successive.

11. Procédé suivant l'une des revendications précédentes, **caractérisé en ce que**, lors de l'enregistrement de la au moins une première image (10) de rayons X, on enregistre plusieurs premières images (10) de rayons X et, lors de la production de l'image (11, 17) de masque, on utilise la même formule pour le mélange de plusieurs premières images (10) de rayons X que lors de la production de la deuxième image (16) successive suivant le stade e) pour le mélange de la première image (14) successive et de la deuxième image (15) de rayons X.

**12.** Procédé suivant l'une des revendications précédentes, **caractérisé en ce que** l'on détermine un changement de position d'un objet médical entre la première image (14) successive et le deuxième image (15) de rayons X, et on mélange des parties respectives d'objet de la première image (14) successive et/ou de la deuxième image (15) de rayons X, en appliquant une compensation de déplacement, dans lequel, dans les parties respectives d'objet, on détecte l'objet médical dans la première image (14) successive et dans la deuxième image (15) de rayons X.

**13.** Appareil (1) médical à rayons X pour effectuer un examen aux rayons X, comprenant

- une unité (20) d'enregistrement constituée :

- pour enregistrer plusieurs premières images (10) de rayons X d'une région du corps dans une première phase (8) de l'examen aux rayons X,
- pour faire la moyenne des plusieurs premières images (10) de rayons X en une image (17) de masque suivant une formule de récursion,
- pour mettre à jour l'image (17) de masque à chaque première image (10) de rayons X arrivée nouvellement,
- pour prescrire l'image (14) de masque mise à jour en dernier de la première phase comme première image (14) successive, et
- pour enregistrer une deuxième image (15) de rayons X de la région du corps dans une deuxième phase de l'examen aux rayons X,

- une unité (21) de mise à disposition pour la mise à disposition de la première image (14) successive de la région du corps,
dans lequel la deuxième image (15) de rayons X représente la région du corps à un instant d'enregistrement plus tardif que la première image (14) successive,
- une première unité (22) de détermination pour la détermination d'une mesure (D) d'écart, qui concerne un écart entre la première image (14) successive et la deuxième image (15) de rayons X,
- une deuxième unité (23) de détermination pour la détermination d'une grandeur (α) d'établissement d'une moyenne en fonction de la mesure (D) d'écart suivant une prescription déterminée à l'avance,
- une unité (24) de production pour la production d'une deuxième image (16) successive à partir de la première image (14) successive ensemble avec le deuxième image (15) de rayons X,
dans lequel par la grandeur (α) d'établissement d'une moyenne, il est prescrit les proportions en lesquelles la première image (14) successive et la deuxième image (15) de rayons X doivent être mélangées par l'unité (24) de production, et
- une unité (25) de regroupement pour la formation d'une image (18) d'ensemble composée de l'image (17) de masque et de la deuxième image (16) successive.

FIG 1

# FIG 2

FIG 3

**IN DER BESCHREIBUNG AUFGEFÜHRTE DOKUMENTE**

**In der Beschreibung aufgeführte Patentdokumente**

- US 20080137935 A1
- US 5091925 A
- US 20090076369 A1
- US 6314160 B1